# FASCICULE DE BREVET EUROPEEN

(11) **EP 0 956 019 B1**
(45) Date de publication et mention de la délivrance du brevet: **15.10.2003**
(21) Numéro de dépôt: 97950237.4
(22) Date de dépôt: 05.12.1997
(51) Int. Cl.: A61K 31/445, A61P 25/00

(54) **NOUVELLE APPLICATION THERAPEUTIQUE D'UN DERIVE DE LA THIENYLCYCLOHEXYLAMINE**
NEUE THERAPEUTISCHE VERWENDUNG EINES THIENYLCYCLOHEXYLAMINDERIVATES
NOVEL THERAPEUTIC APPLICATION OF A THIENYCYCLOHEXYLAMINE DERIVATIVE

(30) Priorité: 06.12.1996 FR 9614996; 13.06.1997 FR 9707361
(43) Date de publication de la demande: 17.11.1999
(73) Titulaire: SOCIETE DE CONSEILS DE RECHERCHES ET D'APPLICATIONS SCIENTIFIQUES (S.C.R.A.S.), 75016 Paris (FR)
(72) Inventeur: LALLEMENT, Guy, F-38180 Seyssins (FR); D'ARBIGNY, Pierre, F-92400 Courbevoie (FR); KAMENKA, Jean-Marc, F-34090 Montpellier (FR)
(74) Mandataire: Bourgouin, André
(86) Numéro de dépôt international: FR9702219
(87) Numéro de publication internationale: WO98024434

(56) Documents cités:
- EP-A- 0 317 953
- WO-A-90/05524

## Description

La présente invention concerne l'utilisation d'une thiénylcyclohexylamine, seule ou en association avec d'autres substances à activité pharmaceutique, pour la préparation d'un médicament destiné à limiter ou inhiber les effets dus à des produits neurotoxiques ou neurotoxiniques. L'invention concerne également un produit comprenant une thiénylcyclohexylamine et au moins une substance anticholinergique, anticonvulsivante ou réactivatrice des cholinestérases, et une composition pharmaceutique le contenant. Ce produit est particulièrement intéressant également pour son activité à limiter ou inhiber les effets dus à des produits neurotoxiques ou neurotoxiniques.

La famille des produits neurotoxiques ou neurotoxiniques comprend les produits tels que les organophosphorés que l'on peut trouver, par exemple, dans les insecticides ou pesticides à usage ménager ou industriel, mais également dans les gaz de combat, comme le soman, le sarin, le tabun ou le VX. Parmi les thérapies existantes telles que la polymédication pour lutter contre l'intoxication de tels composés, aucune ne prévient totalement l'apparition de séquelles neuropathologiques.

L'invention a pour objet l'utilisation de la thiénylcyclohexylamine répondant à la formule 2-méthyl-1-(1-pipéridinyl)-1-(2-thiényl) cyclohexane, pour la préparation d'un médicament destiné à limiter ou inhiber les effets dus à des produits neurotoxiques ou neurotoxiniques chez le primate et notamment chez l'homme. Elle peut être utilisée seule ou en association avec d'autres substances à activité pharmaceutique susceptible de limiter ou inhiber les effets dus à des produits neurotoxiques ou neurotoxiniques.

La thiénylcyclohexylamine telle que définie ci-dessus, est décrite dans le brevet EP 396734 comme agent neuroprotecteur contre le glutamate d'origine endogène. Compte tenu de l'existence de 2 carbones asymétriques, cette thiénylcyclohexylamine peut être sous forme racémique, de diastéréoisomères ou d'énantiomères substantiellement purs.

La préparation des diastéréoisomères de la 1-thiénylcyclohexylamine consiste à faire réagir le 2-bromothiénylmagnésien sur la 2-méthylcyclo hexanone, à traiter le 2-méthyl-1-(2-thiényl) cyclohexanol ainsi obtenu par NaN₃ pour obtenir l'azide correspondante, à réduire cette azide en amine et enfin à traiter avec du 1,5-halogénopentane. Les diastéréoisomères cis et trans sont séparés par chromatographie préparative sur gel de silice, en utilisant un mélange hexane / éther (95/5 en volume) : la première fraction, correspondant au composé trans, cristallise à 40-41° C : la deuxième fraction, correspondant au composé cis, cristallise à 80-81° C. Les énantiomères peuvent être obtenus en utilisant des acides optiquement actifs tels que l'acide di-O.O'-4-toluoyltartrique.

L'invention a particulièrement pour objet l'utilisation de la thiénylcyclohexylamine telle définie ci-dessus, avec au moins une substance choisie parmi les substances anticholinergiques. anticonvulsivantes et réactivatrices des cholinestérases.

L'invention a particulièrement pour objet l'utilisation de la thiénylcyclohexylamine telle que définie ci-dessus, avec au moins une substance anticholinergique, au moins une substance anticonvulsivante et au moins une substance réactivatrice des cholinestérases.

Les termes pharmacologiques utilisés ont la signification classique connue de l'homme de l'art. Ainsi, parmi les substances anticholinergique, on peut citer les substances suivantes: atropine, scopolamine, N-oxyde d'atropine, dihexyvérine et méthylsulfate de tiemonium. Parmi les substances anticonvulsivantes, on peut citer par exemple les substances suivantes : phénobarbital, primidone, carbamazépine, éthosuximide, phénytome, valproate de sodium, progabide, gabapentine, vigabatrine, loprazolam, les benzodiazépines telles que clonazepam, clobazam, diazepam et prodiazepam. Parmi les substances réactivatrices des cholinestérases, on peut citer la pralidoxime, l'obidoxime, le HI6.

L'invention a également pour objet une utilisation de la thiénylcyclohexylamine telle que définie ci-dessus, caractérisée en ce que la thiénylcyclohexylamine est associée à un inhibiteur réversible de la cholinestérase destiné à être administré avant exposition aux produits neurotoxiques ou neurotoxiniques. Parmi les inhibiteurs réversibles de la cholinestérase, on peut citer la pyridostigmine, la physostigmine.

L'invention a également pour objet, à titre de médicament, un produit contenant la thiénylcyclohexylamine telle que définie ci-dessus, associée avec au moins une substance à activité pharmaceutique susceptible de limiter ou d'inhiber les effets dus à des produits neurotoxiques ou neurotoxiniques, ainsi que des compositions pharmaceutiques le contenant.

L'invention a plus particulièrement pour objet, à titre de médicament, un produit contenant la thiénylcyclohexylamine telle que définie ci-dessus. sous forme racémique, de diastéréoisomères ou d'énantiomères substantiellement purs, associée avec au moins une substance choisie parmi les substances anticholinergiques, anticonvuisivantes et réactivatrices des cholinestérases. De préférence, le produit tel que défini ci-dessus, comprend la thiénylcyclohexylamine telle que définie ci-dessus, associée avec au moins une substance anticholinergique, une substance anticonvulsivante et une substance réactivatrice des cholinestérases.

L'invention concerne également un produit comprenant la thiénylcyclohexylamine et au moins une substance choisie parmi les substances anticholinergiques, anticonvulsivantes et réactivatrices des cholinestérases en tant que produit de combinaison pour une utilisation simultanée, séparée ou étalée dans le temps, pour limiter ou inhiber les effets dus à des produits neurotoxiques ou neurotoxiniques.

L'invention a également pour objet un produit tel que défini ci-dessus caractérisé en ce qu'il comprend de plus un inhibiteur de la cholinestérase destiné à être administré avant l'exposition aux produits neurotoxiques ou neurotoxiniques.

La thiénylcyclohexylamine telle que définie ci-dessus, peut être administrée à une dose comprise entre 0.001 et 10 mg/kg, de préférence entre 0,01 et 0,1 mg/kg. Les substances qui lui sont éventuellement associées telles que les subtances anticholinergiques, anticonvulsivantes ou réactivatrices des cholinestérases, connues en pharmacologie, sont administrées aux doses habituellement conseillées dans leurs domaines pharmacologiques respectifs.

La thiénylcyclohexylamine telle définie ci-dessus ainsi que les substances à activité pharmaceutique qui lui sont éventuellement associées, peuvent être administrées par les voies classiques d'administration telle que orale, intramusculaire, intrapéritonéale, sous-cutanée ou intraveineuse. Elles peuvent être administrées simultanément ou séparément, par des voies d'administration identiques ou différentes. De préférence, la thiénylcyclohexylamine est administrée par voie intraveineuse et les substances à activité pharmaceutique qui lui sont éventuellement associées telles que les susbtances anticholinergiques, anticonvulsivantes et réactivatrices des cholinestérases, sont administrées par voie intramusculaire ou intraveineuse. Dans le cas où la thiénylcyclohexylamine est associée à au moins une des substances à activité pharmaceutique telles que définies ci-dessus, l'administration de la thiénylcyclohexylamine peut être différée par rapport à l'administration de ces autres substances.

L'administration de la thiénylcyclohexylamine peut aussi être effectuée à partir du moment de l'intoxication jusqu'à plusieurs heures après cette intoxication. De préférence, cette administration est effectuée dans les deux heures suivant l'intoxication. De préférence encore, cette administration peut être effectuée entre 10 minutes et 45 minutes après intoxication.

Des résultats figurant ci-après dans la partie expérimentale montrent la grande efficacité de l'administration complémentaire de la cis-thiénylcyclohexylamine de 10 à 45 minutes après intoxication.

L'invention a donc pour objet l'utilisation décrite ci-dessus, caractérisée en ce que la 2-méthyl-1-(1-pipéridinyl)-1-(2-thiényl) cyclohexane, sous forme racémique, de diastéréoisomères ou d'énantiomères substantiellement purs, est administrée dans les deux heures et de préférence dans l'heure suivant l'intoxication avec des produits neurotoxiques ou neurotoxiniques.

Les exemples suivants sont présentés pour illustrer les procédures ci-dessus et ne doivent en aucun cas être considérés comme une limite à la portée de l'invention.

### PARTIE EXPÉRIMENTALE :

### Etude pharmacologique

### 1) Première série d'expérimentation :

### Protocole d'étude :

Neuf singes Cynomolgus ont été traités 1 heure avant intoxication par la pyridostigmine (0.2 mg/kg ; i.m.). Cette dose de pyridostigmine inhibe 30 % des cholinestérases plasmatiques, ce qui correspond aux normes de protection retenues dans les pays de l'OTAN. Les animaux sont ensuite intoxiqués par 5 DL₅₀ de soman. composé organophosphoré (30 µg/kg ; i.m.) puis traités 1 minute après intoxication par le "cocktail thérapeutique" : sulfate d'atropine (0.5 mg/kg : i.m.) + valium (0,2 mg/kg ; i.m.) + pralidoxime (30 mg/kg ; i.m.). Ce mélange de 3 drogues correspond à la thérapeutique d'urgence mise en place au niveau des personnels sous forme de seringues autoinjectables.

Dix minutes après intoxication, les animaux reçoivent la cis-thiénylcyclohexylamine par voie i.v. (0,01 ; 0,03 et 0,1 mg/kg, 3 animaux par dose).

Les animaux (non contraints) sont ensuite observés, les signes de toxicité aiguë et de récupération sont notés durant 17 périodes d'observation allant de 2 minutes post intoxication jusqu'à 3 semaines. Trois semaines après intoxication, les animaux sont sacrifiés, le cerveau est prélevé puis après immersion 1 mois dans le formol, on pratique des coupes histologiques (10 µm) colorées au Luxol Fast Blue HE afin de rechercher d'éventuelles séquelles neuropathologiques.

Nous avons réalisé des expérimentations semblables chez des animaux témoins ne recevant pas de cis-thiénylcyclohexylamine. Ainsi, nous avons pu voir l'influence de ce composé sur lcs signes cliniques des animaux intoxiqués et sur les séquelles neuropathologiques.

### Résultats :

### Animaux témoins (n = 4) :

Chez ces animaux, nous notons des signes sévères d'intoxication (fasciculations musculaires, tremblements, mâchonnements) qui apparaissent en 1 à 2 minutes après administration du soman, les animaux ont ensuite des convulsions tonico-cloniques associees a un opisthotonos. Tous les animaux tombent ensuite rapidement dans le coma en 5 minutes environ. Le coma des animaux dure de 20 à 40 minutes. Après cette phase de coma, les animaux récupèrent lentement durant les 6 heures qui suivent l'intoxication, les tremblements cessent progressivement. Un jour après intoxication, tous les animaux sont capables de marcher, d'agripper et de grimper mais ils ne récupèrent une activité normale que 4 jours après l'intoxication. Trois semaines après intoxication, l'examen histopathologique des tissus cérébraux montre une dépopulation neuronale marquée dans la deuxieme couche du cortex frontopariéral chez tous les primates.

### Animaux traités par la thiénylcyclohexylamine (n = 3 par dose) :

L'admimistration i.v. de cis-thiénylcyclohexylamine est pratiquée, comme nous l'avons indique dans le protocole d'étude, durant la phase de coma des animaux. L'observation de l'état clinique est résumée dans le tableau suivant :

| **Dose de la cis-thiényl- cyclohexyl- amine (mg/kg)** | **Signes de toxicité aiguë identiques aux témoins** | **Prévention des troubles respiratoires** | **Arrêt rapide des tremblements** | **Ataxie** | **Délai de récupération (témoins ; 4 jours)** |
|---|---|---|---|---|---|
| 0.1 | X | X | X | X | 4 jours |
| 0,03 | X | X | X | | 4 jours |
| 0,01 | X | | | | 24 heures, pas de rebond à 48 heures |

Trois semaines après l'intoxication, l'examen histologique des tissus cérébraux des animaux traités par thiénylcyclohexylamine montre une densité neuronale normale dans la couche II du cortex frontopariétal, quelle que soit la dose de cis-thiénylcyclohexylamine utilisée. Les comptages effectués dans cette région cérébrale indiquent une différence significative entre la densité neuronale observée chez les animaux témoins et celle des animaux traités par thiénylcyclohexylamine. Il y a donc un effet neuroprotecteur de la thiénylcyclohexylamine qui apparaît dès la dose la plus faible.

### Conclusion :

Dans nos conditions expérimentales, le traitement d'urgence a été administré 1 minute après intoxication. Dans ces conditions, l'état de mal déclenché par le soman ne dure que 3 à 5 minutes. Pourtant, des séquelles neuropathologiques subsistent 3 semaines après intoxication. L'administration de cis-thiénylcyclohexylamine en plus de la polymédication d'urgence permet, à la dose de 0,01 mg/kg i.v., d'améliorer sensiblement la récupération des animaux intoxiqués 48 heures après administration du soman et de prévenir la raréfaction neuronale observée dans le cortex frontopariétal des animaux témoins.

### 2) Deuxième série d'expérimentation :

La série d'expérimentation décrite ci-après est plus proche d'une situation réelle. Le protocole adopté est le suivant :

Des animaux intoxiqués par 8 DL₅₀ ne reçoivent 1 minute après intoxication que l'équivalent d'une seule seringue autoinjectable comprenant du sulfate d'atropine, du valium et du pralidoxime. La cis-thiénylcyclohexylamine est administrée 45 minutes après par voie iv ; ce délai de 45 minutes étant censé correspondre au temps nécessaire au ramassage d'un blessé puis à son acheminement sur un poste de secours où seraient pratiquées une décontamination de ses vêtements par une équipe spécialisée et la pose d'une voie d'injection iv par un personnel médical.

### Protocole d'étude :

Un mois avant l'expérimentation, 6 singes Cynomolgus sont opérés afin de permettre l'implantation d'électrodes corticales pour l'enregistrement EEG selon un protocole standardisé (Mestries et al., Sci. Tech. Anim. Lab., 1984, 9, 35-46). Des soins post-opératoires adaptés (antibiothérapie généralisée pendant 10 jours et application locale d'antiseptique pendant 5 jours) sont pratiqués.

La veille de l'expérimentation, les animaux sont anesthésiés (Imalgène, 3 mg/kg, im) puis placés sur un siège de contention. Vingt-quatre heures après (délai nécessaire pour éliminer l'lmalgène à plus de 99 %), les primates sont connectés à un enregistreur EEG (ALVAR 16 canaux). L'activité EEG des animaux est enregistrée en continu durant 6 heures et une analyse de la répartition d'énergie par bandes de fréquences est effectuée après analyse FFT (bande delta 0,5-5 Hz, bande thêta 5-10 Hz, bande alpha 10-16 Hz, bande bêta 16-48 Hz) afin de permettre le calcul d'un index EEG (% delta + thêta / % bêta). Les animaux sont prétraités pai la pyridostigmine (0,2 mg/kg, im) 1 heure avant intoxication par le soman. Cette dose de pyridostigmine inhibe 30 % des cholinestérases plasmatiques. ce qui correspond aux normes de protection retenues par les pays de l'OTAN. Les animaux sont ensuite intoxiqués par 8 DL₅₀ de soman, composé organophosphoré (30 µg/kg, im) puis traités 1 minute après intoxication par le mélange : sulfate d'atropine (0,25 mg/kg, im) + valium (0,1 mg/kg, im) + pralidoxime (15 mg/kg, im). Ces doses, chez le primate, équivalent à l'administration chez l'homme d'une seule seringue autoinjectable. Les animaux sont ensuite observés et la présence ou l'absence chez chaque animal de 5 signes de toxicité aiguë (tremblements, clonies ou crises tonico-cloniques, coma. troubles respiratoires, hyperréactivité aux stimuli sonores ou tactiles) ainsi que de 4 signes de récupération (réflexe oculaire, réflexe de morsure, agrippement, suivi visuel) sont notés au temps 2, 5, 10, 15, 30, 45 minutes après intoxication.

Quarante-cinq minutes après intoxication, 3 animaux sont traités par la cis-thiénylcyclohexylamine, administrée par voie iv à la dose de 0,1 mg/kg. Les 3 autres animaux ne reçoivent pas de traitement. Les signes de toxicité et de récupération précédemment mentionnés sont ensuite notés pour chaque animal au temps 1 h, 1 h 15, 1 h 30, 1 h 45, 2 h, 2 h 30, 3 h, 3 h 30, 4 h. 4 h 30, 5 h post-intoxication. Cinq heures après intoxication (soit 6 heures depuis le début de l'expérimentation compte tenu du temps de prétraitement par la pyridostigmine), l'enregistrement de l'activité EEG des animaux est arrêté, les primates sont enlevés du siège de contention puis transférés (sans anesthésie) dans une cage de grande dimension. L'état clinique des animaux est ensuite apprécié au temps 5 h, 5 h 30, 6 h puis 24 h, 48 h, 3 jours, 4 jours. 1 semaine, 2 semaines, 3 semaines post-intoxication à partir des mêmes signes de toxicité aiguë et de récupération que ceux précédemment mentionnés auxquels s'ajoutent la présence ou l'absence de prostration (signe de toxicité), ainsi que leur capacité à s'asseoir, à marcher et grimper et à s'alimenter (signes de récupération).

Trois semaines après intoxication, les animaux sont sacrifiés par injection iv de pentobarbital, le cerveau est rapidement prélevé et placé dans du formol à 10 % pendant 1 mois (changement de bain toutes les semaines). A l'issue, l'examen histologique des tissus cérébraux est réalisé après coloration à l'Hemalun-éosine / luxol Fast-Blue.

### Résultats :

### Quarante-cinq premières minutes post-intoxication : (avant administration de la cis-thiénylcyclohexylamine), Période P₁)

### • Signes cliniques :

Chez tous les animaux, nous notons des signes sévères d'intoxication (fasciculations musculaires, tremblements, mâchonnements) qui apparaissent 2 à 3 minutes aprés administration du soman. Tous les animaux ont ensuite des convulsions tonico-cloniques associées à un opisthotonos. La latence des convulsions est d'environ 3 à 4 minutes. Durant cette phase aiguë, les animaux sont cyanosés. Cinq des 6 animaux intoxiqués tombent ensuite dans le coma en 5 à 8 minutes environ. Cette phase de coma dure environ 30 minutes. Des mouvements respiratoires anarchiques (dyspnée) sont observés chez les 6 animaux et de fortes sécrétions sont notées. Quarante-cinq minutes après l'intoxication, 1 animal est encore dans le coma et un seul sur les 6 a récupéré un réflexe palpébral normal et une capacité de suivi visuel. A ce même temps, tous les animaux présentent des tremblements persistants associés à des troubles du rythme respiratoire importants.

### • Activité EEG :

L'apparition des crises tonico-cloniques mentionnées précédemment s'accompagnent d'un état de mal (*status epilepticus*) caractéristique de l'intoxication organophosphorée. Cet état de mal dure environ 5 minutes puis cesse durant la phase de coma, pour réapparaître ensuite (c'est à dire environ 30 minutes après intoxication). L'analyse par bandes de fréquence montre un accroissement de la répartition d'énergie EEG dans la bande bêta associée à une chute de l'énergie relative de la bande delta, aussi bien sur les dérivations temporales que pariétales. Cette augmentation de l'énergie relative dans les hautes fréquences est tout à fait caractéristique de l'injection de diazépam aux animaux (Lipp, Arch. Int. Pharmacodyn., 1973, 202, 244-251). Le calcul de l'index EEG (delta + thêta / bêta), durant les 45 premières minutes post-intoxication, montre une diminution très importante de ce dernier, par rapport à la période précédant l'injection du soman. Ceci traduit, d'après les données de la littérature, une hyperexcitation au niveau cérébral (Nagymajtényi et al., Neurolox. Teratology, 1988, 10, 429-434).

### De la quarante-cinquième minute post-intoxication à la fin de l'enregistrement EEG : (5 heures post-intoxication, Période P₂)

### • Signes cliniques :

Les 3 animaux ne recevant pas de cis-thiénylcyclohexylamine montrent des tremblements persistants associés à des clonies pendant plusieurs heures, ainsi que des troubles du rythme respiratoire associés à des mouvements abdominaux anarchiques. Leur récupération est très lente, puisque 1 seul animal sur 3 présente des réflexes de morsure et d'agrippement ainsi qu'une capacité de suivi visuel 6 heures après intoxication. Les troubles respiratoires associés à une hypersécrétion sont notés durant toute la période P₂. Un des animaux meurt de détresse respiratoire 4 heures après intoxication.

Chez les animaux recevant de la cis-thiénylcyclohexylamine, on note, 5 à 10 minutes après l'injection iv du produit, un arrêt des clonies ou des crises tonico-cloniques, ainsi qu'une disparition totale des troubles respiratoires. Les animaux ont une ventilation régulière. Après la phase initiale de coma, les 3 animaux traités par la cis-thiénylcyclohexylamine récupèrent leurs capacités de morsure, d'agrippement et de suivi visuel, 2 heures à 2 h 30 après intoxication. Aucun des 3 animaux traités par la cis-thiénylcyclohexylamine ne meurt durant les 5 premières heures qui suivent l'injection de soman.

### • Activité EEG :

Chez les animaux ne recevant pas de cis-thiénylcyclohexylamine, on note une persistance du *status epilepticus* durant 2 h 30 à 3 heures après l'intoxication. L'activité EEG diminue ensuite progressivement avec a) - une prédominance de l'énergie relative dans les basses fréquences (bandes delta) associée à b) - une baisse de l'énergie relative dans les hautes fréquences (bandes bêta). Le calcul de l'index EEG durant la période P₂ montre, par rapport à la période précédant l'injection du soman, une forte augmentation de ce dernier, à la fois sur les dérivations temporales et pariétales. Cette augmentation d'index, par accroissement de la part relative de la bande delta et chute de celle de la bande bêta. est prédictive de séquelles neuropathologiques (Philipens et al., Pharmacol. Biochem. Behav., 1992, 42, 711-719).

Chez les animaux recevant de la cis-thiénylcyclohexylamine, on note un arrêt du *status epilepticus* 8 à 10 minutes après l'injection iv de ce produit. Le tracé hautes fréquences / haute énergie laisse la place à un tracé d'ondes lentes (2-4 Hz) caractéristique de la cis-thiénylcyclohexylamine pendant environ 1 heure. Le tracé EEG retourne ensuite progressivement à la normale sans reprise d'activité paroxystique. L'analyse par bande de fréquence sur la période P₂ montre une répartition de l'énergie EEG équivalente à celle enregistrée durant la période précédant l'intoxication. L'index EEG calculé durant cette période est identique à celui de la période pré-intoxication.

### • De cinq heures post-intoxication au sacrifice des animaux : (3 semaines après l'expérimentation)

Les 2 animaux témoins survivants récupèrent très lentement après leur remise en cage. Un de ces 2 animaux meurt 48 heures après l'expérimentation dans un état d'épuisement extrême (l'animal était incapable de se mouvoir et de s'alimenter seul). Le seul animal survivant de la série témoin présente une récupération tout à fait satisfaisante dès la cinquième heure post-intoxication, associant réflexe oculaire, agrippement, morsure, marche, grimper et alimentation.

Chez les 3 animaux traités par la cis-thiénylcyclohexylamine, on note une récupération clinique totale 5 heures après l'intoxication, hormis la capacité de marche et grimper qui ne revient à la normale chez ces animaux que le lendemain de l'expérimentation. Aucun des 3 animaux traités par la cis-thiénylcyclohexylamine ne décède durant les 3 semaines d'observation et leur récupération clinique est tout à fait satisfaisante.

### Examen histopathologique du cerveau des animaux survivants :

L'examen histopathologique du cerveau du seul animal survivant du groupe témoin, non traité par la cis-thiénylcyclohexylamine, montre des séquelles neuropathologiques dans l'hippocampe et dans le cortex frontopariétal. Dans l'hippocampe, on note des foyers de dépopulation neuronale dans la couche pyramidale de l'aire CA₁, ainsi qu'une atteinte sévère de la couche granulaire du gyrus denté. Dans le cortex frontopariétal, on note une raréfaction neuronale de la couche II-III.

Chez les animaux traités par la cis-thiénylcyclohexylamine, on ne détecte aucune lésion pathologique.

### Résumé :

Voir tableau page ci-après.

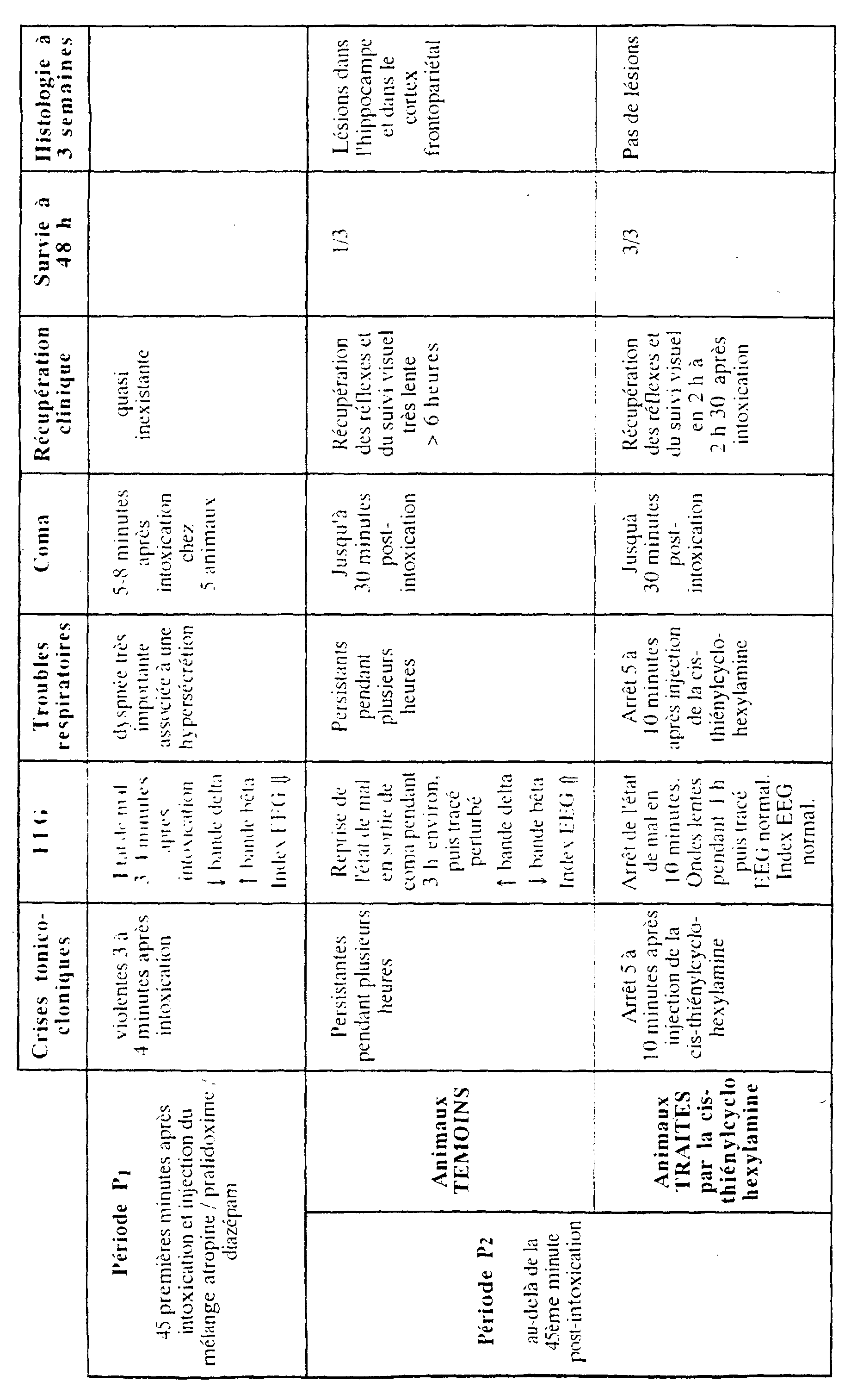

### Conclusion :

Dans cette deuxième série d'expérimentations, réalisée dans des conditions proches d'une situation réelle, nous avons pu montrer que l'administration de la cis-thiénylcyclohexylamine, en complément de la seringue autoinjectable,
a) - améliore très nettement la survie des animaux intoxiqués
b) - favorise leur récupération clinique
c) - normalise rapidement leur activité EEG
d) - prévient totalement la survenue de séquelles neuropathologiques.

Nos résultats mettent donc clairement en évidence le bénéfice thérapeutique lié à l'administration de la cis-thiénylcyclohexylamine chez un sujet gravement intoxiqué qui n'aurait pu s'autoadministrer qu'une seule seringue tricompartiments, et ceci même si l'administration de la cis-thiénylcyclohexylamine n'est pratiquée que 45 minutes après le traitement d'urgence.

### Formulation :

Préparation pour une solution injectable de la cis-thiénylcyclohexylamine
- lyophilisat de la cis-thiénylcyclohexylamine 0.5 mg
- mannitol 25 mg
- chlorure de sodium 85,5 mg
- eau pour préparations injectables : qsp 10,0 ml

Les substances qui sont éventuellement associées à la thiénylcyclohexylamine, sont utilisées sous leurs formes habituelles dans leurs domaines pharmacologiques respectifs.

## Revendications

1. Utilisation de la thiénylcyclohexylamine répondant à la formule 2-méthyl-1-(1-pipéridinyl)-1-(2-thiényl) cyclohexane, sous forme racémique, de diastéréoisomères ou d'énantiomères substantiellement purs, seule ou en association avec d'autres substances à activité pharmaceutique, pour la préparation d'un médicament destiné à limiter ou inhiber les effets dus à l'exposition à des produits neurotoxiques ou neurotoxiniques à l'exclusion du glutamate comme agent neurotoxique.

2. Utilisation de la thiénylcyclohexylamine répondant à la formule 2-méthyl-1-(1-pipéridinyl)-1-(2-thiényl) cyclohexane, sous forme racémique, de diastéréoisomères ou d'énantiomères substantiellement purs, seule ou en association avec d'autres substances à activité pharmaceutique, pour la préparation d'un médicament destiné à limiter ou inhiber les effets dus à l'exposition à des produits neurotoxiques ou neurotoxiniques sélectionnés parmi les insecticides et les gaz de combat.

3. Utilisation selon la revendication 2, **caractérisée en ce que** les gaz de combat sont choisis parmi le soman, le surin, le tabun et le VX.

4. Utilisation selon l'une des revendications 1 à 3 **caractérisée en ce que** la thiénylcyclohexylamine est associée avec une ou plusieurs autres substances à activité pharmaceutique susceptible de limiter ou inhiber les effets dus à des produits neurotoxiques ou neurotoxiniques.

5. Utilisation selon la revendication 4, **caractérisée en ce que** la thiénylcyclohexylamine est associée avec au moins une substance choisie parmi les substances anticholinergiques, anticonvulsivantes et réactivatrices des cholinestérases.

6. Utilisation selon l'une des revendications 4 à 5, **caractérisée en ce que** la thiénylcyclohexylamine est associée avec au moins une substance anticholinergique, au moins une substance anticonvulsivante et au moins une substance réactivatrice des cholinestérases.

7. Utilisation selon l'une des revendications 5 à 6, **caractérisée en ce que** la substance anticholinergique est choisie parmi l'atropine, la scopolamine, le N-oxyde d'atropine, la dihexyvérine et le méthylsulfate de tiémonium .

8. Utilisation selon l'une des revendications 5 à 6, **caractérisée en ce que** la substance anticonvulsivante est choisie parmi les substances suivantes : phénobarbital, primidone, les benzodiazépines, carbamazépine, éthosuximide, phénytoïne, valproate de sodium, progabide, gabapentine, vigabatrine et loprazolam.

9. Utilisation selon la revendication 8, **caractérisée en ce que** la substance anticonvulsivante est une benzodiazépine choisie parmi clonazepam, clobazam, diazepam et prodiazepam.

10. Utilisation selon l'une des revendications 5 à 6, **caractérisée en ce que** la substance réactivatrice des cholinestérases est choisie parmi les substances suivantes : la pralidoxime, l'obidoxime et le HI6.

11. Utilisation selon l'une des revendications 1 à 10, **caractérisée en ce que** la thiénylcyclohexylamine est associée à un inhibiteur réversible de la cholinestérase destiné à être administré avant exposition aux produits neurotoxiques ou neurotoxiniques.

12. Utilisation selon l'une des revendications 1 à 11, **caractérisée en ce que** l'inhibiteur réversible de la cholinestérase est choisi parmi la pyridostigmine, la physostigmine.

13. Utilisation suivant l'une quelconque des revendications 1 à 12, **caractérisée en ce que** la 2-méthyl-1-(1-pipéridinyl)-1-(2-thiényl) cyclohexane, sous forme racémique, de diastéréoisomères ou d'énantiomères substantiellement purs, est administrée dans les deux heures et, de préférence, dans l'heure suivant l'intoxication avec des produits neurotoxiques ou neurotoxiniques.

14. Composition pharmaceutique contenant, à titre de principe actif, un produit contenant la thiénylcyclohexylamine répondant à la formule 2-méthyl-1-(1-pipéridinyl)-1-(2-thiényl) cyclohexane, sous forme racémique, de diastéréoisomères ou d'énantiomères substantiellement purs, associée avec au moins une substance à activité pharmaceutique susceptible de limiter ou inhiber les effets dus à l'exposition de produits neurotoxiques ou neurotoxiniques.

15. Composition pharmaceutique selon la revendication 14, **caractérisée en ce que** la thiénylcyclohexylamine est associée avec au moins une substance choisie parmi les substances anticholinergiques, anticonvulsivantes et réactivatrices des cholinestérases.

16. Composition pharmaceutique selon l'une des revendications 14 à 15, **caractérisée en ce que** la thiénylcyclohexylamine est associée avec au moins une substance anticholinergique, au moins une substance anticonvulsivante et au moins une substance réactivatrice des cholinestérases.

17. Composition pharmaceutique selon l'une des revendications 15 à 16, **caractérisée en ce que** la substance anticholinergique est choisie parmi l'atropine, la scopolamine, l'atropine N-oxyde, la dihexyvérine et le tiémonium méthylsulfate.

18. Composition pharmaceutique selon l'une des revendications 15 à 16, **caractérisée en ce que** la substance anticonvulsivante est choisie parmi les substances suivantes: phénobarbital, primidone, carbamazépine, éthosuximide, phénytoïne, valproate de sodium, progabide, gabapentine, vigabatrine, loprazolam et les benzodiazépines telles clonozepam, clobazam, diazepam et prodiazepam.

19. Composition pharmaceutique selon l'une des revendications 15 à 16, **caractérisée en ce que** la substance réactivatrice des cholinestérases est choisie parmi la pralidoxime, l'obidoxime, le HI6.

20. Produit comprenant la thiénylcyclohexylamine et au moins une substance choisie parmi les substances anticholinergiques, anticonvulsivantes et réactivatrices des cholinestérases en tant que produit de combinaison pour une utilisation simultanée, séparée ou étalée dans le temps pour limiter ou inhiber les effets dus à l'exposition de produits neurotoxiques ou neurotoxiniques.

21. Produit selon la revendication 20, **caractérisé en ce qu'**il comprend de plus un inhibiteur de la cholinestérase destiné à être administré avant l'exposition au produit neurotoxique ou neurotoxinique.

22. Produit selon la revendication 21, **caractérisé en ce que** l'inhibiteur réversible de la cholinestérase est choisi parmi la pyridostigmine, la physostigmine.

## Patentansprüche

1. Verwendung von Thienylcyclohexylamin, das der Formel 2-Methyl-1-(1-piperidinyl)-1-(2-thienyl)cyclohexan entspricht, in racemischer Form, in Form von im wesentlichen reinen Diastereoisomeren oder Enantiomeren, allein oder in Kombination mit anderen Substanzen mit pharmazeutischer Wirkung, für die Herstellung eines Medikaments, das dafür bestimmt ist, die Wirkungen zu begrenzen oder zu hemmen, die durch Aussetzung an neurotoxische Produkte oder Neurotoxine, unter Ausschluß von Glutamat als neurotoxisches Mittel, verursacht werden.

2. Verwendung von Thienylcyclohexylamin, das der Formel 2-Methyl-1-(1-piperidinyl)-1-(2-thienyl)cyclohexan entspricht, in racemischer Form, in von Form von im wesentlichen reinen Diastereoisomeren oder Enantiomeren, allein oder in Kombination mit anderen Substanzen mit pharmazeutischer Wirkung, für die Herstellung eines Medikaments, das dazu bestimmt ist, die Wirkungen zu begrenzen oder zu hemmen, die durch Aussetzen an neurotoxische Produkte oder Neurotoxine verursacht werden, die aus den Insektiziden und Kampfgasen ausgewählt sind.

3. Verwendung nach Anspruch 2, **dadurch gekennzeichnet, daß** die Kampfgase aus Soman, Sarin, Tabun und VX ausgewählt sind.

4. Verwendung nach einem der Ansprüche 1 bis 3, **dadurch gekennzeichnet, daß** das Thienylcyclohexylamin mit einer oder mehreren anderen Substanzen mit pharmazeutischer Wirkung kombiniert ist, die die Wirkungen einer Aussetzung an neurotoxische Produkte oder Neurotoxine begrenzen oder hemmen können.

5. Verwendung nach Anspruch 4, **dadurch gekennzeichnet, daß** das Thienylcyclohexylamin mit mindestens einer Substanz kombiniert ist, die aus den anticholinergen, antikonvulsiven und die Cholinesterasen reaktivierenden Substanzen ausgewählt ist.

6. Verwendung nach einem der Ansprüche 4 bis 5, **dadurch gekennzeichnet, daß** das Thienylcyclohexylamin mit mindestens einer anticholinergen Substanz, mit mindestens einer antikonvulsiven Substanz und mit mindestens einer die Cholinesterasen reaktivierenden Substanz kombiniert ist.

7. Verwendung nach einem der Ansprüche 5 bis 6, **dadurch gekennzeichnet, daß** die anticholinerge Substanz aus Atropin, Scopolamin, Atropin-N-Oxid, Dihexyverin und Tiemoniummethylsulfat ausgewählt ist.

8. Verwendung nach einem der Ansprüche 5 bis 6, **dadurch gekennzeichnet, daß** die antikonvulsive Substanz aus den folgenden Substanzen ausgewählt ist: Phenobarbital, Primidon, Benzodiazopine, Carbamazepin, Ethosuximid, Phenytoin, Natriumvalproat, Progabid, Gabapentin, Vigabatrin und Loprazolam.

9. Verwendung nach Anspruch 8, **dadurch gekennzeichnet, daß** die antikonvulsive Substanz ein Benzodiazepin ist, das aus Clonazepam, Clobazam, Diazepam und Prodiazepam ausgewählt ist.

10. Verwendung nach einem der Ansprüche 5 bis 6, **dadurch gekennzeichnet, daß** die die Cholinesterasen reaktivierende Substanz aus den folgenden Substanzen ausgewählt ist: Pralidoxim, Obidoxim und H16.

11. Verwendung nach einem der Ansprüche 1 bis 10, **dadurch gekennzeichnet, daß** das Thienylcyclohexylamin mit einem reversiblen Cholinesterase-Hemmer kombiniert ist, der dazu bestimmt ist, vor Aussetzung an die neurotoxischen Produkte oder Neurotoxine verabreicht zu werden.

12. Verwendung nach einem der Ansprüche 1 bis 11, **dadurch gekennzeichnet, daß** der reversible Cholinesterase-Hemmer aus Pyridostigmin, Physostigmin ausgewählt ist.

13. Verwendung nach einem der Ansprüche 1 bis 12, **dadurch gekennzeichnet, daß** das 2-Methyl-1-(1-piperidinyl)-1-(2-thienyl)cyclohexan in racemischer Form, in Form von im wesentlichen reinen Diastereoisomeren oder Enantiomeren innerhalb von zwei Stunden und vorzugsweise innerhalb einer Stunde nach der Intoxikation mit neurotoxischen Produkten oder Neurotoxinen verabreicht wird.

14. Pharmazeutische Zusammensetzung, die als Wirkstoff ein Produkt enthält, das Thienylcyclohexylamin, das der Formel 2-Methyl-1-(1-piperidinyl)-1-(2-thienyl)cyclohexan entspricht, in racemischer Form, in Form von im wesentlichen reinen Diastereoisomeren oder Enantiomeren enthält, das mit mindestens einer Substanz mit pharmazeutischer Wirkung kombiniert ist, die die Wirkungen der Aussetzung an neurotoxische Produkte oder Neurotoxine begrenzen oder hemmen kann.

15. Pharmazeutische Zusammensetzung nach Anspruch 14, **dadurch gekennzeichnet, daß** das Thienylcyclohexylamin mit mindestens einer Substanz kombiniert ist, die aus den anticholinergen, antikonvulsiven und die Cholinesterasen reaktivierenden Substanzen ausgewählt ist.

16. Pharmazeutische Zusammensetzung nach einem der Ansprüche 14 bis 15, **dadurch gekennzeichnet, daß** das Thienylcyclohexylamin mit mindestens einer anticholinergen Substanz, mit mindestens einer antikonvulsiven Substanz und mindestens einer die Cholinesterasen reaktivierenden Substanz kombiniert ist.

17. Pharmazeutische Zusammensetzung nach einem der Ansprüche 15 bis 16, **dadurch gekennzeichnet, daß** die anticholinerge Substanz aus Atropin, Scopolamin, Atropin-N-oxid, Dihexyverin und Tiemoniummethylsulfat ausgewählt ist.

18. Pharmazeutische Zusammensetzung nach einem der Ansprüche 15 bis 16, **dadurch gekennzeichnet, daß** die antikonvulsive Substanz aus den folgenden Substanzen ausgewählt ist: Phenobarbital, Primidon, Carbamazepin, Ethosuximid, Phenytoin, Natriumvalproat, Progabid, Gabapentin, Vigabatrin und Loprazolam und Benzodiazepine, wie z.B. Clonazepam, Clobazam, Diazepam und Prodiazepam.

19. Pharmazeutische Zusammensetzung nach einem der Ansprüche 15 bis 16, **dadurch gekennzeichnet, daß** die die Cholinesterasen reaktivierende Substanz aus Pralidoxim, Obidoxim, H16 ausgewählt ist.

20. Produkt, umfassend Thienylcyclohexylamin und mindestens eine aus den anticholinergen, antikonvulsiven und die Cholinesterasen reaktivierenden Substanzen ausgewählte Substanz als Kombinationsprodukt für eine gleichzeitige, getrennte oder zeitlich verteilte Verwendung zur Begrenzung oder Hemmung der Wirkungen einer Aussetzung an neurotoxische Produkte oder Neurotoxine.

21. Produkt nach Anspruch 20, **dadurch gekennzeichnet, daß** es außerdem einen Cholinesterase-Hemmer umfaßt, der dazu bestimmt ist, vor der Aussetzung an das neurotoxische Produkt oder Neurotoxin verabreicht zu werden.

22. Produkt nach Anspruch 21, **dadurch gekennzeichnet, daß** der reversible Cholinesterase-Hemmer aus Pyridostigmin und Physostigmin ausgewählt ist.

## Claims

1. Use of thienylcyclohexylamine corresponding to the formula 2-methyl-1-(1-piperidinyl)-1-(2-thienyl) cyclohexane, in substantially pure racemic, diastereoisomeric or enantiomeric form, by itself or in combination with other pharmaceutically active substances, for the preparation of a medicament intended to limit or inhibit the effects due to neurotoxic or neurotoxinic products excluding glutamate as neurotoxic agent.

2. Use of thienylcyclohexylamine corresponding to the formula 2-methyl-1-(1-piperidinyl)-1-(2-thienyl) cyclohexane, in substantially pure racemic, diastereoisomeric or enantiomeric form, by itself or in combination with other pharmaceutically active substances, for the preparation of a medicament intended to limit or inhibit the effects due to neurotoxic or neurotoxinic products selected from insecticides or poison gas for use in warfare.

3. Use according to claim 2, **characterized in that** poison gas for use in warfare is chosen from soman, sarin, tabun and VX.

4. Use according to one of claims 1 to 3, **characterized in that** the thienylcyclohexylamine is combined with one or more other pharmaceutically active substances capable of limiting or inhibiting the effects due to neurotoxic or neurotoxinic products.

5. Use according to claim 4, **characterized in that** the thienylcyclohexylamine is combined with at least one substance chosen from anticholinergic, anticonvulsive substances and substances which reactivate cholinesterases.

6. Use according to one of claims 4 to 5, **characterized in that** the thienylcyclohexylamine is combined with at least one anticholinergic substance, at least one anticonvulsive substance and at least one substance which reactivates cholinesterases.

7. Use according to one of claims 5 to 6, **characterized in that** the anticholinergic substance is chosen from atropine, scopolamine, atropine N-oxide, dihexyverine and tiemonium methylsulphate.

8. Use according to one of claims 5 to 6, **characterized in that** the anticonvulsive substance is chosen from the following substances: phenobarbital, primidone, the benzodiazepins, carbamazepine, ethosuximide, phenytoin, sodium valproate, progabide, gabapentin, vigabatrin and loprazolam.

9. Use according to claim 8, **characterized in that** the anticonvulsive substance is a benzodiazepin chosen from clonazepam, clobazam, diazepam and prodiazepam.

10. Use according to one of claims 5 to 6, **characterized in that** the substance which reactivates cholinesterases is chosen from the following substances: pralidoxime, obidoxime and HI6.

11. Use according to one of claims 1 to 10, **characterized in that** the thienylcyclohexylamine is combined with a reversible cholinesterase inhibitor intended to be administered before exposure to the neurotoxic or neurotoxinic products.

12. Use according to one of claims 1 to 11, **characterized in that** the reversible cholinesterase inhibitor is chosen from pyridostigmine, physostigmine.

13. Use according any one of claims 1 to 12, **characterized in that** 2-methyl-1-(1-piperidinyl)-1-(2-thienyl) cyclohexane, in substantially pure racemic, diastereoisomeric or enantiomeric form, is administered within two hours and, preferably, within the hour following intoxication with neurotoxic or neurotoxinic products.

14. Pharmaceutical composition containing, as active ingredient, a product containing thienylcyclohexylamine corresponding to the formula 2-methyl-1-(1-piperidinyl)-1-(2-thienyl) cyclohexane, in substantially pure racemic, diastereoisomeric or enantiomeric form, combined with at least one pharmaceutically active substance capable of limiting or inhibiting the effects due to neurotoxic or neurotoxinic products.

15. Pharmaceutical composition according to claim 14, **characterized in that** the thienylcyclohexylamine is combined with at least one substance chosen from anticholinergic, anticonvulsive substances and substances which reactivate cholinesterases.

16. Pharmaceutical composition according to one of claims 14 to 15, **characterized in that** the thienylcyclohexylamine is combined with at least one anticholinergic substance, at least one anticonvulsive substance and at least one substance which reactivates cholinesterases.

17. Pharmaceutical composition according to one of claims 15 to 16, **characterized in that** the anticholinergic substance is chosen from atropine, scopolamine, atropine N-oxide, dihexyverine and tiemonium methylsulphate.

18. Pharmaceutical composition acording to one of claims 15 to 16, **characterized in that** the anticonvulsive substance is chosen from the following substances: phenobarbital, primidone, carbamazepine, ethosuximide, phenytoin, sodium valproate, progabide, gabapentin, vigabatrin, loprazolam and the benzodiazepins such as clonozepam, clobazam, diazepam and prodiazepam.

19. Pharmaceutical composition according to one of claims 15 to 16, **characterized in that** the substance which reactivates cholinesterases is chosen from pralidoxime, obidoxime, HI6.

20. Product containing thienylcyclohexylamine and at least one substance chosen from anticholinergic, anticonvulsive substances and substances which reactivate cholinesterases as a combination product for simultaneous or separate use, or use spread out over time to limit or inhibit the effects due to neurotoxic or neurotoxinic products.

21. Product according to claim 20, **characterized in that** it contains a cholinesterase inhibitor intended to be administered before exposure to the neurotoxic or neurotoxinic product.

22. Product according to claim 21, **characterized in that** the reversible cholinesterase inhibitor is chosen from pyridostigmine, physostigmine.
